# EUROPEAN PATENT SPECIFICATION

(11) **EP 2 671 570 B1**
(45) Date of publication and mention of the grant of the patent: **30.05.2018**
(21) Application number: 12741665.9
(22) Date of filing: 31.01.2012
(51) Int. Cl.: A61K 9/16, A61K 47/50, A61K 47/02, A61K 49/08, A61K 49/18, C01G 45/12, G01N 33/58

(54) **MAGNETIC NANOPARTICLE, HAVING A CURIE TEMPERATURE WHICH IS WITHIN BIOCOMPATIBLE TEMPERATURE RANGE, AND METHOD FOR PREPARING SAME**
MAGNETISCHE NANOPARTIKEL MIT EINER CURIE-TEMPERATUR INNERHALB EINES BIOKOMPATIBLEN TEMPERATURBEREICHS UND VERFAHREN ZU IHRER HERSTELLUNG
NANOPARTICULE MAGNÉTIQUE AYANT UNE TEMPÉRATURE DE CURIE QUI EST DANS LA PLAGE DES TEMPÉRATURES BIOCOMPATIBLES, ET SON PROCÉDÉ DE PRÉPARATION

(30) Priority: 31.01.2011 KR 20110009824
(43) Date of publication of application: 11.12.2013
(73) Proprietor: Korea University Research and Business Foundation, Seoul 136-713 (KR)
(72) Inventor: KIM, Young Keun, Seoul 135-906 (KR); WU, Jun Hua, Seoul 136-701 (KR); MIN, Ji Hyun, Seoul 151-015 (KR); LEE, Ji-Sung, Seoul 136-074 (KR); JUNG, Jae-Ho, Suwon-si Gyeonggi-do 443-370 (KR)
(74) Representative: Eisenführ Speiser
(86) International application number: PCT/KR2012/000739
(87) International publication number: WO 2012/105794

(56) References cited:
- CN-A- 101 173 167
- KR-A- 20070 058 358
- KR-A- 20070 088 391
- KR-A- 20090 119 867
- KR-A- 20100 030 264
- KR-B1- 100 652 251
- US-A1- 2005 249 817
- US-A1- 2007 258 888
- VASSEUR S ET AL: "Lanthanum manganese perovskite nanoparticles as possible in vivo mediators for magnetic hyperthermia", JOURNAL OF MAGNETISM AND MAGNETIC MATERIALS, ELSEVIER SCIENCE PUBLISHERS, AMSTERDAM, NL, vol. 302, no. 2, 1 July 2006 (2006-07-01), pages 315-320, XP024984479, ISSN: 0304-8853, DOI: 10.1016/J.JMMM.2005.09.026 [retrieved on 2006-07-01]
- KAMAN O ET AL: "Silica encapsulated manganese perovskite nanoparticles for magnetically induced hyperthermia without the risk of overheating", NANOTECHNOLOGY, IOP, BRISTOL, GB, vol. 20, no. 27, 8 July 2009 (2009-07-08), page 275610, XP020160674, ISSN: 0957-4484
- HAM WOO SEUNG ET AL: "Microstructure and Magnetic Properties of LaSrMnO Nanoparticles and Their Application to Cardiac Immunoassay", IEEE TRANSACTIONS ON MAGNETICS, IEEE SERVICE CENTER, NEW YORK, NY, US, vol. 51, no. 11, 1 November 2015 (2015-11-01), pages 1-4, XP011587389, ISSN: 0018-9464, DOI: 10.1109/TMAG.2015.2438021 [retrieved on 2015-10-23]
- JAEMOON YANG ET AL: "Antibody conjugated magnetic PLGA nanoparticles for diagnosis and treatment of breast cancer", JOURNAL OF MATERIALS CHEMISTRY, vol. 17, no. 26, 1 January 2007 (2007-01-01), page 2695, XP055449425, GB ISSN: 0959-9428, DOI: 10.1039/b702538f

## Description

### [Technical Field]

The invention is defined in the claims.

The present invention relates to magnetic nanoparticles having a Curie temperature within a biocompatible temperature range. Also described are methods for preparing the same, nanocomposites for target substance detection comprising the same, and methods for obtaining an image of a living body or specimen.

### [Background Art]

Detection of a biological control agent using a magnetic nanoparticle is easy to use and causes relatively less damage to a detected cell, and thus is a subject of much interest. Recent studies have aimed to increase a magnetization value of a magnetic nanoparticle in order to increase sensitivity of biological control agent detection based on magnetic properties. In addition to a detection apparatus using a magnetic nanoparticle, a detection apparatus using biotin-avidin bonding is often used for biological control agent detection, and signal amplification, yet this detection apparatus has many non-specific responses and high signal noise.

CN 101 173 167 discloses a magnetic material comprising lanthanum, calcium or strontium and manganese oxide, which has a Curie temperature around room temperature and may be used as a sensor since its emissivity changes around room temperature.

In J. Magn. Magn. Mater. 2006, 302(2), 315, Vasseur et al. describe nanoparticles having the formula La_{0.75}Sr_{0.25}MnO₃. The magnetization and the Curie temperature decreases with decreasing crystallite size and the nanoparticels can be used for magnetic resonance imaging.

Kaman et al. disclose in Nanotechnology, 2009, 20, 275610, silica encapsulated manganese perovskite nanoparticles, which can be used in cancer treatment for magnetically induced hyperthermia.

Meanwhile, the most problematic issue in applying a magnetic nanoparticle in the domain of bio-medical technology is agglomeration of the magnetic nanoparticles. When the magnetic nanoparticle is used in a living body, agglomeration causes precipitation in blood vessels, triggering thrombosis, and thereby the magnetic nanoparticle's outer surface area decreases and efficiency of a
magnetic nano-based diagnosis/drug delivery/medicine may decrease. In particular, in a case of a diagnosis system based on a magnetic nanoparticle used in vitro, agglomeration of the magnetic nanoparticles interferes with biochemical reactions such as an antigen-antibody reaction, causing an increase in signal noise, and thus diagnosis efficiency may decrease.Accordingly, there is a great need to develop a magnetic nanoparticle which can decrease non-specific responses and increase signal detection sensitivity, thereby increasing a ratio of signal to noise (signal purification).

### [Disclosure]

### [Technical Problem]

The present disclosure is directed to providing magnetic nanoparticles having a Curie temperature within a biocompatible temperature range, methods for preparing the same, nanocomposites and compositions for target substance detection comprising the same, and methods for obtaining an image of a living body or specimen.

### [Technical Solution]

Disclosed herein is a magnetic nanoparticle having a Curie temperature within the temperature range of -80°C to 41°C, comprising a rare earth metal, a divalent metal, and a transition metal oxide.

Further disclosed is a method for preparing a magnetic nanoparticle as disclosed herein, comprising (a) a step of reducing a precursor of the rare earth metal, a precursor of the divalent metal, and a precursor of the transition metal oxide, thereby forming the magnetic nanoparticle; and (b) a step of heat treating the magnetic nanoparticle.

Still further disclosed is a nanocomposite comprising a magnetic nanoparticle as disclosed herein; and a biological control agent attached to a surface of the magnetic nanoparticle.

The present invention is defined in the claims. It provides a composition for target substance detection comprising a magnetic nanoparticle as disclosed herein or a nanocomposite as disclosed herein; and a magnet-antibody composite, as defined in the claims.

Further disclosed is a method for obtaining an image of a living body or specimen, comprising a step of administering a composition for target substance detection according to the present invention to a living body or specimen; and a step of sensing a signal transmitted by a magnetic nanoparticle or nanocomposite from the living body or specimen, thereby obtaining the image.

### [Advantageous Effects]

As the magnetic nanoparticle disclosed herein has a Curie temperature within the temperature range of 0°C to 41°C, the ferromagnetic and paramagnetic properties of the magnetic nanoparticle may be controlled within a biocompatible temperature range at which a biological control agent is not destroyed, and the temperature of the magnetic nanoparticle is adjusted to control the magnetic properties thereof such that the properties of the magnetic nanoparticle may be used only when ferromagnetic properties are required, such as in a case of signal amplification in detecting, separating, and delivering biological control agents. Accordingly, the magnetic nanoparticle disclosed herein may minimize adverse effects of ferromagnetic properties, and may be used in the effective detection and separation of biological control agents.

### [Description of Drawings]

FIG. 1 illustrates a nanocomposite as disclosed herein.
FIG. 2 illustrates a magnetic nanoparticle as disclosed herein to which a detection means is attached.
FIG. 3 illustrates a nanocomposite as disclosed herein to which a detection means is attached.
FIG. 4 is a schematic diagram showing a process of detecting a target substance using a composition for target substance detection as disclosed herein.
FIG. 5 is a picture of a high-resolution transmission electron microscope (TEM) of a magnetic nanoparticle as disclosed herein.
FIG. 6 is a graph of a X-ray diffraction (XRD) pattern of a magnetic nanoparticle as disclosed herein.
FIG. 7 is a graph of magnetization value versus temperature (M-T) of a magnetic nanoparticle as disclosed herein.

### Brief Description of Elements in the Drawings

1,2: nanocomposite / 10,25: magnetic nanoparticle
11: biological control agent / 12,26: detection means
20: substrate / 21: target substance
22: impurities / 23: antibody
24: magnet / 27: massive composite

### [Modes of the Invention]

Hereinafter, exemplary embodiments will be described in detail. The following exemplary embodiments are described in order to enable those of ordinary skill in the art to embody and practice the invention.

It will be understood that, although the terms first, second, etc. may be used herein to describe various elements, these elements should not be limited by these terms. These terms are only used to distinguish one element from another. For example, a first element could be termed a second element, and similarly, a second element could be termed a first element. As used here, the term "and/or" includes any and all combinations of one or more of the associated listed items.

Disclosed herein are magnetic nanoparticles having a Curie temperature within the temperature range of -80°C to 41°C, comprising a rare earth metal, a divalent metal, and a transition metal oxide.

Hereinafter, a magnetic nanoparticle will be described in detail.

A magnetic nanoparticle as disclosed herein has a Curie temperature within the temperature range of -80°C to 41°C, preferably the temperature range of 0°C to 41°C, and more preferably the temperature range of 10°C to 41°C, comprising a rare earth metal, a divalent metal, and a transition metal oxide. The expression "Curie temperature" as used herein may denote a critical temperature where a ferromagnet loses its magnetic properties due to an increase in temperature, and the ferromagnet exhibits paramagnetic properties at a temperature equal to and above the Curie temperature.

As the magnetic nanoparticle disclosed herein has a Curie temperature within a biocompatible temperature range, ferromagnetic and paramagnetic properties of the magnetic nanoparticle may be controlled within a temperature range at which a biological control agent is not destroyed.

An average diameter of a magnetic nanoparticle as disclosed herein is not particularly limited, and may be, for instance, 1 nm to 500 nm, preferably 10 nm to 300 nm, and more preferably 20 nm to 100 nm.

Further, a shape of a magnetic nanoparticle as disclosed herein is not particularly limited, and may be, for instance, spherical, linear, cylindrical, flat, or any combination thereof.

Examples of the rare earth metal as disclosed herein include Sc, Y, La, Ce, Pr, Nd, Pm, Sm, Eu, Gd, Tb, Dy, Ho, Er, Tm, Yb, and Lu, preferably lanthanum metals, such as La, Ce, Pr, Nd, Pm, Sm, Eu, Gd, Tb, Dy, Ho, Er, Tm, Yb, and Lu, and more preferably La and Nd.

Examples of the divalent metal as disclosed herein include Be, Mg, Ca, Sr, Ba, Ra, Pb, V, Nb, Ta, Zn, Cd, and Hg, preferably alkali earth metals, such as Be, Mg, Ca, Sr, Ba, and Ra; and Pb, and more preferably Sr, Ba, Ca, and Pb.

Examples of the transition metal oxide as disclosed herein include oxides of at least one metal selected from the group consisting of Ti, V, Cr, Mn, Fe, Co, Ni, Cu, Zn, Zr, Nb, Mo, Tc, Ru, Rh, Pd, Ag, Cd, Hf, Ta, W,
Re, Os, Ir, Pt, Au, and Hg, and preferably manganese oxide.

The magnetic nanoparticle may comprise 0.5 to 1 molar fraction of the rare earth metal and 0.01 to 0.5 molar fraction of the divalent metal, relative to 1 molar fraction of the transition metal oxide. In the magnetic nanoparticle as disclosed herein, a Curie temperature of the magnetic nanoparticle may be adjusted to the range of -80°C to 41°C by controlling the molar fraction of each component within the above-described ranges.

The magnetic nanoparticle as disclosed herein may form a structured body together with another material for an additional function. Types of the structured body are not particularly limited, and may be, for instance, a core-shell structure, a dumbbell structure, a cluster structure, a thin layer structure, an alloy structure, a multi-layered nanowire, or any combination thereof. Herein, the other materials constituting the structured body together with the magnetic nanoparticle may be a silica, a ceramic material, an organic material, a metallic material, a magnetic material, a polymer, or a semiconductor material, depending on the purpose of use.

In the case of the core-shell structureas disclosed herein, a magnetic nanoparticle disclosed herein may form a core part while the above other material forms a shell part surrounding the core part. Alternatively, the above other material may form a core part while a magnetic nanoparticle disclosed herein forms a shell part. Herein, the shell part of the core-shell structure may have pores, and thus it can be a porous core-shell form. In the case of the porous core-shell as disclosed herein, a drug can be supported thereon, and thus it can be used as a drug delivering body.

In the case of the dumbbell structureas disclosed herein , one part of the dumbbell may be formed with a magnetic nanoparticle
as disclosed herein, while the other part is formed with another material, such as a magnetic material, a metallic material, a polymer, a ceramic and semiconductor material, depending on the purpose of use.

In the case of the multi-layered nanowire structure as disclosed herein, the nanowire may have a multi-layered structure wherein a magnetic nanoparticle as disclosed herein and another material, such as gold (Au), are alternately formed.

In the case of the thin layer structureas disclosed herein , a magnetic nanoparticle as disclosed herein may form a thin layer, and a layered structure can be formed with the thin layer made of a magnetic nanoparticle as disclosed herein and another thin layer made of another material.

Furthermore, the structured body consisting of a complex combination of the above structures, such as a structured body wherein one-dimensional nanowire structures are projected from a thin layer structure or a structured body wherein spherical nanoparticles are attached to a nanowire, may be used depending on the purpose of use.

Further disclosed herein are methods for preparing a magnetic nanoparticle, comprising (a) a step of reducing a precursor of the rare earth metal, a precursor of the divalent metal, and a precursor of the transition metal oxide, thereby forming a magnetic nanoparticle; and (b) a step of heat treating the magnetic nanoparticle.

For preparing a magnetic nanoparticle as disclosed herein, a step of dissolving the precursor of the rare earth metal, the precursor of the divalent metal, the precursor of the transition metal oxide, and a reducing agent in a solvent,
heating to a temperature in the range of 80°C to 130°C, and uniformly mixing for 1 to 2 hours at said temperature may be conducted prior to step (a).

Types of the precursor of the rare earth metal as disclosed herein are not particularly limited, and include the aforementioned rare earth metals as well as anything that can become the rare earth metal by reduction through an oxidation-reduction reaction. Examples of the precursor of the rare earth metal as disclosed herein include lanthanum acetylacetonate (La(acac)₂) and lanthanum nitrate(La(NO₃)₃6H₂O), preferably lanthanum acetylacetonate.

Types of the precursor of the divalent metal as disclosed herein are not particularly limited, and include the aforementioned divalent metals as well as anything that can become the divalent metal by reduction through an oxidation-reduction reaction. Examples of the precursor of the divalent metal as disclosed herein include strontium acetylacetonate (Sr(acac)₃) and strontium acetate (Sr(CH₃COO)₂), preferably strontium acetylacetonate.

Types of the precursor of the transition metal oxide as disclosed herein are not particularly limited, and include the aforementioned transition metals as well as anything that can become the transition metal oxide by reduction through an oxidation-reduction reaction. Examples of the precursor of the transition metal oxide as disclosed herein include manganese acetylacetonate (Mn(acac)₃) and manganese acetate (Mn(CH3COO)₂·4H₂O), preferably manganese acetylacetonate.

Molar fractions of the precursor of the rare earth metal, the precursor of the divalent metal, and the precursor of the transition metal oxide, as disclosed herein, are the same as described above.

The reducing agent helps to reduce each of the precursor of the rare earth metal, the precursor of the divalent metal, and the precursor of the transition metal oxide through an oxidation-reduction reaction so that the rare earth metal, the divalent metal, and the transition metal oxide can be agglomerated into a single nanoparticle.

Types of the reducing agent are not particularly limited, and anything can be used without limitation as long as it can reduce all of the precursor of the rare earth metal, the precursor of the divalent metal, and the precursor of the transition metal oxide. Examples of the reducing agent as disclosed herein include 1,2-hexadecanediol.

A content of the reducing agent is not particularly limited, and can be properly selected within the scope being able to reduce all of the precursor of the rare earth metal, the precursor of the divalent metal, and the precursor of the transition metal oxide.

Types of the solvents are not particularly limited, and anything can be used without limitation as long as it can dissolve the precursor of the rare earth metal, the precursor of the divalent metal, the precursor of the transition metal oxide, and the reducing agent. Examples of the solvent as disclosed herein include alkylethers having an alkyl group with 1 to 12 carbon atoms, arylethers having an aryl group with 6 to 18 carbon atoms, aralkylethers with 7 to 21 carbon atoms, and alkenylethers having an alkenyl group with 2 to 12 carbon atoms.

A content of the solvent as disclosed herein is not particularly limited, and can be properly selected within the scope of being able to disolve all of the precursor of the rare earth metal, the precursor of the divalent metal, the precursor of the transition metal oxide, and the reducing agent.

When the heating temperature is less than 80°C in the preparation step of the mixed solution as disclosed herein, the mixing of the components in a solvent may not be uniform, and when exceeding 130°C, the precursor or reducing agent may react in advance. Further, uniform mixing of each component in the mixed solution can be achieved by controlling the time for which the heating temperature is maintained within the above-described range.

In the preparation step of the mixed solution as disclosed herein, a surfactant may be further dissolved in a solvent along with the precursor of the rare earth metal, the precursor of the divalent metal, the precursor of the transition metal oxide, and the reducing agent.

In a case in which the surfactant is further dissolved in the preparation step of the mixed solution as disclosed herein, dispersibility of the magnetic nanoparticle in an aqueous solution as well as an affinity to a biological control agent can be increased.

Types of the surfactant as disclosed herein are not particularly limited, and any material can be used as long as it shows amphipathy. Examples of the surfactant as disclosed herein include polyalkyleneglycol, polyetherimide, polyvinylpyrrolidone, hydrophilic vinyl polymer,
and copolymers of at least two of the aforementioned.

When the copolymer is used, the copolymer as disclosed herein can preferably be a block copolymer of polyethylene glycol (PEG)-polypropylene glycol (PPG)-polyethylene glycol (PEG) or a block copolymer of polyethylene oxide (PEO)-polypropylene oxide (PPO)-polyethylene oxide (PEO).

In a method for preparing a magnetic nanoparticle as disclosed herein, a step of reducing the precursor of the rare earth metal, the precursor of the divalent metal, and the precursor of the transition metal can be performed after preparing the mixed solution, as described above. The precursor components and the reducing agent contained in the mixed solution undergo an oxidation-reduction reaction such that the reducing agent are oxidized while the precursors are reduced to become the rare earth metal, the divalent metal, and the transition metal oxide.

In particular, a step of the reduction as disclosed herein may be performed by heating the mixed solution to a temperature in the range of 220°C to 300°C, and maintaining the temperature for 1 to 2 hours. When the heating temperature is less than 220°C in the reduction step, the oxidation-reduction reaction between the precursor components and the reducing agent may not be sufficient, and when exceeding 300°C, agglomeration of the nanoparticles may occur. Further, a smooth reduction of each precursor component can be achieved by controlling the time for which the heating temperature is maintained within the above-described range.

The method for preparing the magnetic nanoparticle as disclosed herein may be performed by a step of reducing each precursor component contained in the mixed solution to the rare earth metal, the divalent metal, and the transition metal oxide, and forming the magnetic nanoparticle by cooling it.

When each precursor component contained in the mixed solution is reduced to the rare earth metal, the divalent metal, and the transition metal oxide, and cooled as described above, the rare earth metal, the divalent metal, and the transition metal oxide may agglomerate during the cooling process, thereby forming nano-sized particles. The cooling temperature as disclosed herein is not particularly limited and can be any temperature at which the nano-sized particles can be formed, and the cooling can preferably be conducted to a room temperature.

The methods for cooling the mixed solution as disclosed herein are not particularly limited, and any conventional means in the art can be used without limitation.

In the method for preparing the magnetic nanoparticle as disclosed herein, step (a) can be performed under an inert gas atmosphere, such as an argon gas atmosphere. Unexpected oxidation of the precursor components or the magnetic nanoparticle can be prevented by performing step (a) under an inert gas atmosphere.

The method for preparing the magnetic nanoparticle as disclosed herein may further comprise a step of washing the magnetic nanoparticle formed in step (a) using centrifugation and magnetic separation after step (a).

In particular, after step (a), anhydrous ethanol can be added to the magnetic nanoparticle, and centrifugation and magnetic separation may be performed to remove remaining precursor components and reducing agent, thereby separating the magnetic nanoparticle only.

The method for preparing the magnetic nanoparticle as disclosed herein may comprise (b) a step of heat treating the magnetic nanoparticle. In the method for preparing the magnetic nanoparticle as disclosed herein, crystallinity of the magnetic nanoparticle can be increased by performing step (b), thereby enabling preparation of the magnetic nanoparticle having a Curie temperature within the range of 0°C to 41°C.

In the method for preparing the magnetic nanoparticle as disclosed herein, step (b) may be performed by heating the magnetic nanoparticle to a temperature in the range of 300°C to 1000°C in a heating furnace, and maintaining the temperature for 1 to 13 hours. Types of the heating furnace are not particularly limited, and any means which is conventionally used in the art can be used. An exemplary heating furnace as disclosed herein is a ceramic container. When the heating temperature in the heat treatment step is less than 300°C, a heat treatment effect may not be sufficient, and when exceeding 1000°C, a production cost may increase due to excessive energy consumption. In addition, a time for which the heating temperature is maintained is preferably 2 to 12 hours, and by controlling as such, crystallinity of the magnetic nanoparticle can be increased.

In the method for preparing the magnetic nanoparticle as disclosed herein, step (b) may be performed in the heating furnace filled with an inert gas, such as argon gas and nitrogen gas, in order to control a degree of oxidation of the magnetic nanoparticle.

In addition, in the method for preparing the magnetic nanoparticle as disclosed herein, step (b) may be performed in a heating furnace in which an external magnetic field is applied in order to control magnetic properties of the magnetic nanoparticle. Types of the external magnetic field are not parti-cularly limited, any magnetic field which is conventionally used in the art can be used without limitation, and also, a strength of the external magnetic field can be properly selected according to requirements.

The method for preparing the magnetic nanoparticle as disclosed herein may further comprise, prior to step (b), a step of coating the magnetic nanoparticle with a coating material in order to prevent calcination of the magnetic nanoparticle caused by a performance of step (b). Types of the coating material to coat the magnetic nanoparticle are not particularly limited, and preferably a ceramic material, or a semiconductor material such as zinc oxide, magnesium oxide or aluminum oxide, can be used.

The methods as disclosed herein for coating the magnetic nanoparticle with the coating material are not particularly limited, any means which is conventionally used in the art can be used, but preference is given to use of thermal decomposition.

When the step of coating the magnetic nanoparticle with the coating material prior to step (b) is intended to be performed, after step (b), a treatment with an acidic or basic solution may be conducted to remove the coating material covering the magnetic nanoparticle, and after washing, the magnetic nanoparticle as disclosed herein can be separated using a method such as centrifugation.

The method for preparing the magnetic nanoparticle as disclosed herein may further comprise, prior to step (b), a step of filling the magnetic nanoparticle in a nano-template as another means to prevent calcination of the magnetic nanoparticle caused by a performance of step (b). When the magnetic nanoparticle prepared in step (a) is filled in a nano-template and introduced into a heating furnace where the heat treatment is conducted, calcination of the magnetic nanoparticle during the heat treatment can be prevented. The method for filling the magnetic nanoparticle prepared in step (a) in the nano-template may be, for instance, the method described in Korean patent application No. 10-2004-0084468.

When the step of filling the magnetic nanoparticle in the nano-template prior to step (b) is performed as described above, after step (b), the nano-template can be dissolved using a chromic acid solution or a sodium hydroxide solution, thereby extracting the magnetic nanoparticle only.

The method for preparing the magnetic nanoparticle as disclosed herein may further comprise a process of separating a partially calcinated magnetic nanoparticle with a laser treatment or an ultrasonic wave treatment in order to remove the partially calcinated magnetic nanoparticle which may be produced in step (b).

Further disclosed herein are nanocomposites comprising a magnetic nanoparticle as disclosed herein; and a biological control agent attached to a surface of the magnetic nanoparticle.

The details as to the magnetic nanoparticle to be contained in the nanocomposite as disclosed herein are the same as described above.

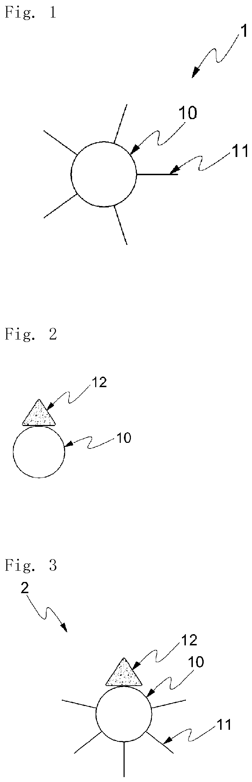

The appended FIG. 1 illustrates a nanocomposite as disclosed herein. As illustrated in FIG. 1, a nanocomposite (1) as disclosed herein may comprise a magnetic nanoparticle (10) and a biological control agent (11) which is attached to a surface of the magnetic nanoparticle (10).

Types of the biological control agent as disclosed herein, attached to a surface of the magnetic nanoparticle are not particularly limited,
and preferably an antigen, an antibody, a protein, or a biocompatible polymer can be used.

Types of the antigen, the antibody, and the protein as disclosed herein are not particularly limited, and anything can be used without limitation as long as it can be conventionally used for target substance detection.

Introducing the antigen, the antibody, and the protein in the surface of a magnetic nanoparticle as disclosed herein can be performed by methods well-known in the art. For instance, the antigen, the antibody, and the protein can be introduced by coating gold (Au) on a surface of a magnetic nanoparticle as disclosed herein, and then introducing thiol groups on a surface of the gold coating, or the antigen, the antibody, and the protein can be introduced by attaching a biocompatible polymer on a surface of a magnetic nanoparticle as disclosed herein by a method to be described below, and then bonding a functional group existing on an end part of the biocompatible polymer with a particular functional group.

The antigen, the antibody, and the protein attached to a surface of a magnetic nanoparticle as disclosed herein may be used for detection and separation of target substance, such as detection and quantification of a target protein.

The biocompatible polymer attached to a surface of the magnetic nanoparticle as disclosed herein can increase dispersibility of the magnetic nanoparticles in aqueous solution and affinity to the biological control agent.

Types of the biocompatible polymer as disclosed herein are not particularly limited, and any material can be used as long as it shows amphipathy. Examples of the biocompatible polymer as disclosed herein include polyalkyleneglycol, polyetherimide, polyvinylpyrrolidone, hydrophilic vinyl polymer, and copolymers of at least two of the aforementioned.

When a copolymer is used as the biocompatible polymer, the copolymer as disclosed herein can preferably be a block copolymer of polyethylene glycol (PEG)-polypropylene glycol (PPG)-polyethylene glycol (PEG) or a block copolymer of polyethylene oxide (PEO)-polypropylene oxide (PPO)-polyethylene oxide (PEO).

The method for introducing the biocompatible polymer to a surface of a magnetic nanoparticle as disclosed herein is not particularly limited, and for instance, the magnetic nanoparticles to whose surface the biocompatible polymer is attached may be prepared, in step (a) of the method for preparing a magnetic nanoparticle as disclosed herein, by dissolving the biocompatible polymer along with the precursor of the rare earth metal, the precursor of the divalent metal, the precursor of the transition metal oxide, and the reducing agent, thereby preparing a mixed solution, and performing step (b) in the same manner.

Upon preparing the nanocomposite as disclosed herein, a stabilizer, such as oleylamine (C₉H₁₈=C₉H₁₇NH₂) and oleic acid (C₉H₁₈=C₈H₁₅COOH), may be added to a solvent.

An aspect of the present invention concerns a composition for target substance detection comprising a magnetic nanoparticle as disclosed herein, or a nanocomposite as disclosed herein, and a magnet-antibody composite, as defined in the claims.

The details of the magnetic nanoparticle or the nanocomposite contained in the composition for target substance detection of the present invention are the same as described above.

A detection means may be attached to a surface of the magnetic nanoparticle, or the nanocomposite to be contained in the composition for target substance detection of the present invention,.

The appended FIG. 2 illustrates the magnetic nanoparticle disclosed herein to whose surface a detection means is attached. As illustrated in FIG. 2, a detection means (12) is attached to a surface of the magnetic nanoparticle (10) disclosed herein, and this can be used as the composition for target substance detection.

The appended FIG. 3 illustrates the nanocomposite disclosed herein to whose surface a detection means is attached. As illustrated in FIG. 3, a detection means (12) is attached to a surface of the nanocomposite (2) disclosed herein, and this can be used as the composition for target substance detection.

According to one embodiment of the present invention, the composition for target substance detection may be used for detecting a particular antigen, such as a particular protein or a particular cell, or an amount thereof, like in an ELISA method or a Western blot method.

The composition for target substance detection of the present invention can form a bond with the target substance by an antibody in the magnet-antibody composite through an antigen-antibody reaction when the target substance is present.

In particular, when a target substance, an antigen, is fixed on a substrate and the composition for target substance detection of the present invention comprising the magnet-antibody composite which may cause an antigen-antibody reaction with the target substance is covered thereon, a single composite consisting of target substance-antibody-magnet can be formed through an antigen-antibody reaction of the target substance and the antibody part of the magnet-antibody composite.

In addition, when the magnetic nanoparticle or the nanocomposite to whose surface a detection means is attached is maintained at a temperature equal to or above a Curie temperature, it loses magnetic properties, and thus is not agglomerated but rather uniformly dispersed in the composition. However, when the composite consisting of target substance-antibody-magnet is formed, the magnetic nanoparticle or the nanocomposite returns to a ferromagnet by controlling the temperature to be equal to or less than a Curie temperature, and thus agglomeration may occur due to an attractive force with a magnet part of the composite consisting of target substance-antibody-magnet.

Herein, when the composition for target substance detection is washed, an individual magnetic nanoparticle or nanocomposite which is not agglomerated with the composite consisting of target substance-antibody-magnet may be removed.

Accordingly, a massive composite consisting of target substance-antibody-magnet-magnetic nanoparticle-detection means, or a massive composite consisting of target substance-antibody-magnet-nanocomposite-detection means can be formed.

The detection means in the massive composite can transmit a particular signal depending on its type, thus enabling detection of a target substance. In a part where the target substance is present, the particular signal can be observed, while in a part where the target substance is not present, the particular signal cannot be observed.

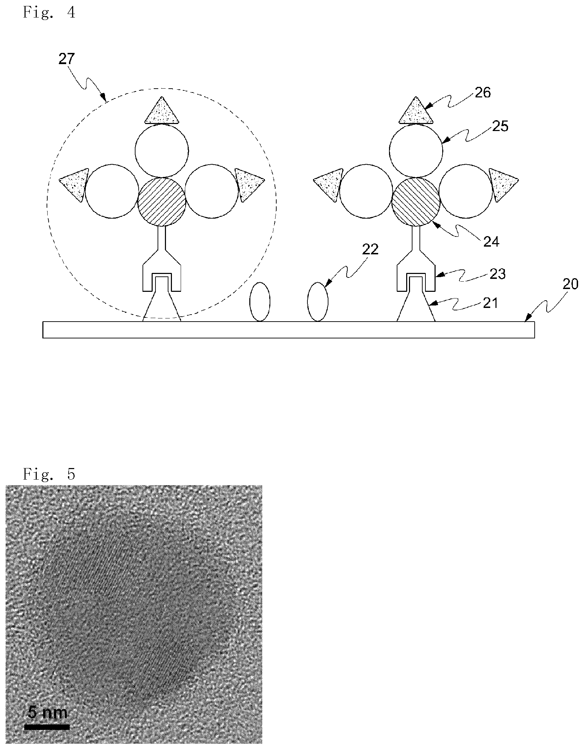

The appended FIG. 4 is a schematic diagram showing a process of detecting a target substance using a composition for target substance detection according to one embodiment of the present invention. As illustrated in FIG. 4, when a target substance (21) is fixed on substrate (20), the target substance (21) and an antibody (23) undergo an antigen-antibody reaction, thereby forming the composite consisting of target substance (21)-antibody (23)-magnet (24). Herein, when the temperature is maintained at equal to or above a Curie temperature of the magnetic nanoparticle of the present invention, a magnetic nanoparticle (25) to whose surface a detection means (26) is attached may lose magnetic properties, and thus agglomeration of the magnetic nanoparticles does not occur and a well-dispersed form is achieved. However, when the temperature is lowered to less than a Curie temperature of the magnetic nanoparticle disclosed herein, the magnetic nanoparticle (25) re-gains ferromagnetic properties and can be agglomerated due to an attractive force with the magnet (24). Thus, a massive composite (27) consisting of target substance (21)-antibody (23)-magnet (24)-magnetic nanoparticle (25)-detection means (26) fixed on substrate (20) can be formed. When a container comprising the composition for target substance detection is washed, components other than the massive composite (27), such as the magnetic nanoparticle to which a detection means is attached, can be removed. In the case of the massive composite (27) fixed on a substrate as described above, a particular signal can be transmitted through the detection means (26), and thus presence of the target substance can be confirmed. Furthermore, in a case of impurities (22) which cannot undergo an antigen-antibody reaction with an antibody (23), the particular signal cannot be observed therein as the massive composite (27) cannot be formed.

The composition for target substance detection of the present invention may form the massive composite through specific bonding with a target substance and control of the magnetic properties of the magnetic nanoparticle, thereby increasing a ratio of signal to noise (signal purification). In other words, the composition for target substance detection of the present invention can increase both specificity and sensitivity to the target substance.

In the composition for target substance detection of the present invention, the detection means is not particularly limited, and any detection means may be used without limitation as long as it can be used in imaging of a living body. In the present invention, examples of the detection means include a fluorescent material and a quantum dot.

In the present invention, when the fluorescent material is used as the detection means, confirmation of a target substance, quantitative analysis, and separation can be performed through a fluorescent image. In the present invention, types of the fluorescent material are not particularly limited, and examples thereof include rhodamine and its derivatives, fluorescein and its derivatives, coumarin and its derivatives, acridine and its derivatives, pyrene and its derivatives, erythrosine and its derivatives, eosin and its derivatives, and 4-acetamido-4'-isothiocyanatostilbene-2,2'-disulfonic acid. Further particular examples of the fluorescent material which can be used in the present invention are as follows.

Examples of **the rhodamine and its derivatives** include 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, sulforhodamine B, sulforhodamine 101, sulfonyl chloride derivatives of sulforhodamine 101 (Texas Red), N,N,N',N'-tetramethyl-6-carboxyrhodamine (TAMRA), tetramethyl rhodamine, tetramethyl rhodamine isothiocyanate (TRITC), riboflavin, rosolic aicd, terbium chelate derivatives, Alexa derivatives, Alexa-350, Alexa-488, Alexa-547, and Alexa-647;
examples of **the fluorescein and its derivatives** include 5-carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein, fluorescein isothiocyanate, QFITC (XRITC), fluorescamine, IR144, IR1446, malachite green isothiocyanate, 4-methylumbelliferone, ortho-cresolphthalein, nitrotyrosine, pararosaniline, phenol red, B-phycoerythrin, and o-phthaldialdehyde;
examples of **the coumarin and its derivatives** include coumarin, 7-amino-4-methylcoumarin (AMC, coumarin 120), 7-amino-4-trifluoromethylcoumarin (coumarin 151), cyanocin, 4'-6-diamidino-2-phenylindole (DAPI), 5',5"-dibromopyrogallol-sulfonephthalein (Bromopyrogallol Red), 7-diethylamino-3-(4'-isothiocyanatophenyl)-4-methylcoumarin diethylenetriamine pentacetate, 4-(4'-diisothiocyanatodihydro-stilbene-2,2'-disulfonic acid, 4,4'-diisothiocyanatostilbene-2,2'-disulfonic acid, 5-[dimethylamino]naphthalene-1-sulfonyl chloride (DNS, dansyl chloride), 4-(4'-dimethylaminophenylazo)benzoic acid (DABCYL), and 4-dimethylaminophenylazophenyl-4'-isothiocyanate (DABITC);
examples of **the acridine and its derivatives** include acridine, acridine isothiocyanate, 5-(2'-aminoethyl)aminonaphthalene-1-sulfonic acid (EDANS), 4-amino-N-[3-vinylsulfonyl)phenyl]naphthalimide-3,5disulfonate (LuciferYellow VS), N-(4-anilino-1-naphthyl)maleimide, anthranilamide, and Brilliant Yellow;
examples of **the pyrene and its derivatives** include pyrene, pyrene butyrate, succinimidyl 1-pyrene butyrate, and Reactive Red 4 (Cibacron Brilliant Red 3B-A);
examples of **the erythrosine and its derivatives** include erythrosin B, erythrosin isothiocyanate, and ethidium;
examples of **the eosin and its derivatives** include eosin, and eosin isothiocyanate; and
**4-acetamido-4'-isothiocyanatostilbene-2,2'disulfonic acid.**

In the present invention, when a quantum dot is used as the detection means, detection of a target substance, quantitative analysis and separation can be performed through a fluorescent image. The quantum dot may have a structure consisting of a center part, a shell part surrounding the center part, and a polymer coating layer coated on the shell part. In the present invention, types of the quantum dot are not particularly limited, and anything may be used without limitation as long as it has biocompatibility and can be used for imaging of a living body. As the components consisting of the center part of the quantum dot, cadmium selenide (CdSe), cadmium telluride (CdTe), cadmium sulfide (CdS), zinc selenide (ZnSe), zinc oxide (ZnO), or zinc sulfide (ZnS) can be mainly used.

In the magnet-antibody composite to be contained in the composition for target substance detection of the present invention, types of the magnet are not particularly limited, and anything can be used without limitation as long as it has magnetic properties. In the present invention, for example, a magnetic nanoparticle as disclosed herein or a conductive material can be used as the magnet.

Types of the conductive material as disclosed herein are not particularly limited, and examples thereof are a metallic material, a magnetic material, and a magnetic alloy. Further examples of the conductive material which can be used in the present invention are as follows.

Examples of the metallic material include Pt, Pd, Ag, Cu, and Au, examples of the magnetic material include Co, Mn, Fe, Ni, Gd, and Mo, and examples of the magnetic alloy include CoCu, CoPt, FePt, CoSm, NiFe, and NiFeCo.

In addition, in the magnet-antibody composite as disclosed herein, types of the antibody are not particularly limited, and anything may be used without limitation as long as it can be bonded to the target substance described below through an antigen-antibody reaction.

Types of the target substance to be detected using the composition for target substance detection of the present invention are not particularly limited, and can be, for instance, at least one selected from the group consisting of a protein, a DNA, and a RNA. In the present invention, types of the protein, the DNA, and the RNA are not particularly limited, and examples thereof can be made to a tumor marker or a bio-marker which is conventionally used in the art.

In the present invention, a protein, the target substance, can be at least one selected from the group consisting of prostate specific antigen (PSA), carcinoembryonic antigen (CEA) MUC1, alpha fetoprotein (AFP), carbohydrate antigen 15-3 (CA 15-3), carbohydrate antigen 19-9 (CA 19-9), carbohydrate antigen 125 (CA 125), free prostate specific antigen (PSAF), prostate specific antigen-a 1-anticymotrypsin comple (PSAC), prostatic acid phosphatase (PAP), human thyroglobulin (hTG), human chorionic gonadotropin beta (HCGb), ferritin (Ferr), neuron specific enolase (NSE), interleukin 2 (IL-2), interleukin 6 (IL-6), beta 2 macroglobulin (B2M), and alpha 2 macroglobulin (A2M).

PSA, PSAF, PSAC, A2M, and PAP are useful tumor markers in the selection of prostate cancer, CEA is a useful tumor marker in the selection of gastrointestinal cancer as a glycoprotein, MUC1 is a tumor marker expressed in ovarian cancer, breast cancer, myeloma, colon cancer, uterine cancer, pancreatic cancer, rectal cancer, and lung cancer, CA 15-3 is a tumor marker expressed in lung cancer, pancreatic cancer, breast cancer, ovarian cancer, and liver cancer, CA 19-9 is a tumor marker expressed in lung cancer, ovarian cancer, liver cancer, and colon cancer, CA 125 is a tumor marker expressed in lung cancer, pancreatic cancer, breast cancer, ovarian cancer, liver cancer, colon cancer, and uterine cancer, hTG is a tumor marker expressed in thyroid cancer and Wilm's tumor, HCGb is a tumor marker expressed in lung cancer, pancreatic cancer, kidney cancer, ovarian cancer, liver cancer, brain cancer, and bladder cancer, Ferr is a tumor marker expressed in lung cancer and brain cancer, NSE is a tumor marker expressed in lung cancer, thyroid cancer, and Wilm's cancer, IL-2 is a tumor marker expressed in kidney cancer, and multiple myeloma, IL-6 is a tumor marker expressed in kidney cancer, breast cancer, ovarian cancer, and multiple myeloma, and B2M is a tumor marker expressed in kidney cancer, ovarian cancer, prostate cancer, and multiple myeloma.

In the present invention, the DNA, the RNA, and the target substance are not particularly limited, and any gene may be used without limitation as long as it is the gene of a virus which invokes infectious disease. In the present invention, examples of the DNA and the RNA include a gene of AIDS virus, a gene of hepatitis B virus, a gene of hepatitis C virus, a gene of malaria virus, a gene of novel swine-origin influenza virus, or a gene of syphilis virus.

Further disclosed herein is a method for obtaining an image of a living body or specimen, comprising a step of administering a composition for target substance detection according to the present invention to the living body or specimen; and a step of sensing a signal transmitted by the nanocomposite from the living body or specimen, thereby obtaining the image.

An expression "specimen", as disclosed herein, may denote a tissue or cell which is separated from the subject to be diagnosed.

Further, the step of administering the composition for target substance detection of the present invention to a living body or specimen can be performed through a path which is conventionally used in the domain of pharmaceuticals, preferably parenteral administration, such as an administration through an intravenous, intraabdominal, intramuscular, subcutaneous, or topical path. In the step of obtaining the image as disclosed herein, magnetic resonance imaging (MRI) and optical imaging are preferably used in order to sense the signal transmitted by a fluorescent material or quantum dot.

The expression "magnetic resonance imaging apparatus", as disclosed herein, may denote an imaging apparatus into which a living body is introduced, energy is absorbed in an atomic nucleus, such as hydrogen, in a tissue of the living body by electromagnetic irradiation at a particular frequency so that a high-energy state is created, then the energy of the atomic nucleus, such as hydrogen, is released after irradiation, and the energy is transformed into a signal which is in turn processed to yield an image. A type of the magnetic resonance imaging apparatus as disclosed herein is not particularly limited, and can be, for instance, a T2 spin-spin relaxation magnetic resonance imaging apparatus. Meanwhile, a co-focal microscope, a fluorescence microscope or an optical equipment for a living body can be used for imaging.

In the method for obtaining an image of a living body or specimen disclosed herein, the composition for target substance detection is administered to a living body or specimen, and thereby the composite consisting of target substance-antibody-magnet can be formed by an antigen-antibody reaction with a particular antigen which is a target substance. Thereafter, when the
temperature of a magnetic nanoparticle as disclosed herein is maintained to be below a Curie temperature using a magnetocaloric effect, a massive composite of target substance-antibody-magnet-nanocomposite-detection means can be formed through ferromagnetic properties of the magnetic nanoparticle as described above. In this case, the massive composites comprising a detection means are distributed in a high concentration around a particular antigen, and thus an amplified image signal can be easily obtained. The magnetocaloric effect is a phenomenon of gradually getting colder or hotter due to a quick transition of a magnetization status of the magnetic material within an external magnetic field, and is well-known in the art.

Hereinafter, Examples of the present invention will be described in detail. The following Examples are described in order to enable those of ordinary skill in the art to embody and practice the present invention.

### EXAMPLES

### Example 1

A magnetic nanoparticle as disclosed herein was prepared by an improved nano-emulsion method based on thermal decomposition as described below.

### (1) Preparation of a mixed solution

0.45 mmol of lanthanum acetylacetonate (La(acac)₃, available from Aldrich), a precursor of rare earth metal, 0.15 mmol of strontium acetylacetonate (Sr(acac)₂, available from Aldrich), a precursor of divalent metal, 0.6 mmol of manganese acetylacetonate (Mn(acac)₃, available from Aldrich), a precursor of transition metal oxide, and 0.1294 g of 1,2-hexadecanediol (available from Aldrich), a reducing agent, were introduced to a container comprising 15 mℓ of dioctylether (available from Wako) under an argon gas atmosphere and dissolved. Thereafter, the solution was heated to 100°C and uniformly stirred for 1.5 hours at 100°C to result in the mixed solution.

### (2) Reduction of the precursors contained in the mixed solution

Thusly-prepared mixed solution was heated to 280°C and maintained for 1.5 hours at 280°C to reduce lanthanum acetylacetonate, strontium acetylacetonate, and manganese acetylacetonate to lanthanum metal (La), strontium metal (Sr), and manganese oxide (MnO₃), respectively, through an oxidation-reduction reaction with 1,2-hexadecanediol.

### (3) Formation of a magnetic nanoparticle

The mixed solution in which all the precursor components were reduced as described above was cooled down to room temperature, thereby forming a magnetic nanoparticle (LaSrMnO₃) in which lanthanum metal, strontium metal, and manganese oxide were agglomerated. An average diameter of the magnetic nanoparticle was about 30 nm.

### (4) Washing the magnetic nanoparticle using centrifugation and magnetic separation

The formed magnetic nanoparticle was added to anhydrous ethanol, and washed with centrifugation and magnetic separation, thereby removing impurities.

### (5) Heat treatment of the magnetic nanoparticle

The washed magnetic nanoparticle was introduced into a ceramic container, heated to 800°C, and maintained at 800°C for 12 hours to perform heat treatment.

### Example 2

The nanocomposite illustrated in the appended FIG. 1 was prepared in the same manner as Example 1, except that, during the process of preparing the (1) mixed solution, 0.1576 g of a block copolymer of polyethylene glycol-polypropylene glycol-polyethylene glycol (available from Aldrich), a biocompatible polymer, was further dissolved in 15 mℓ of dioctylether (available from Wako), the solvent.

### Experimental Example 1

In order to measure a shape of the magnetic nanoparticle prepared in Example 1, the magnetic nanoparticle prepared in Example 1 was dispersed in hexane and dropped on carbon-supported copper grids to prepare a sample for TEM measurement. Thereafter, TEM (Tecnai F20, available from FEI) and energy-dispersive X-ray spectroscopy (EDS) were used to observe the sample. The appended FIG. 5 is a picture of a high-resolution TEM of a magnetic nanoparticle as disclosed herein. As illustrated in FIG. 5, a scale bar denotes 5 nm and the magnetic nanoparticle of Example 1 showed an average diameter of about 30 nm.

### Experimental Example 2

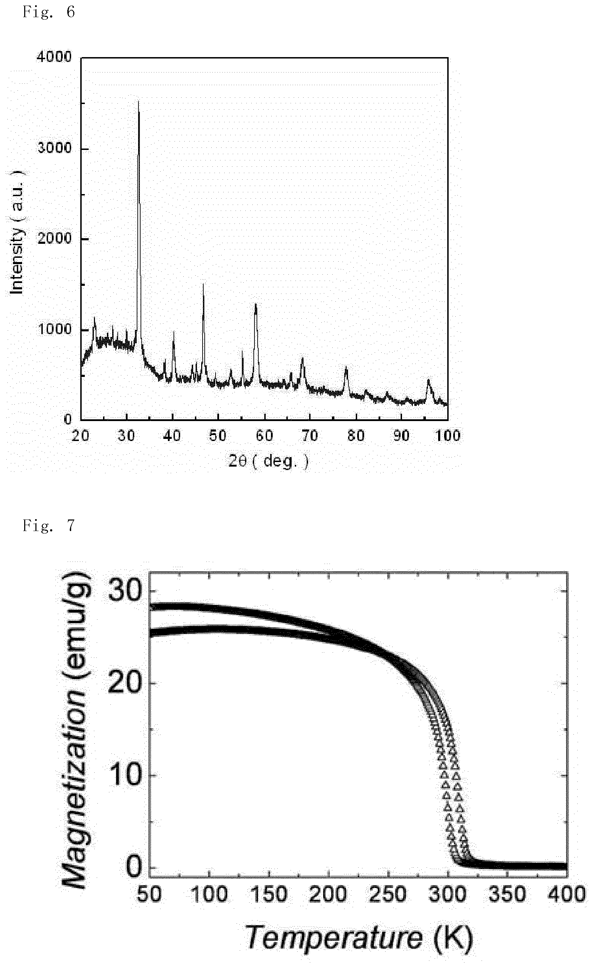

In order to analyze a structure of the magnetic nanoparticle prepared in Example 1, X-ray diffraction analysis of the sample prepared in Experimental Example 1 was performed using an X-ray diffractometer. The appended FIG. 6 is a graph showing an X-ray diffraction (XRD) pattern of a magnetic nanoparticle as disclosed herein. As illustrated in FIG. 6, the magnetic nanoparticle as disclosed herein showed superior crystallinity.

### Experimental Example 3

In order to measure magnetic properties of the magnetic nanoparticle prepared in Example 1, a change of magnetization value of the sample prepared in

Experimental Example 1 in accordance with temperature was measured using a vibrating sample magnetometer (VSM, VSM 7300, available from Lakeshore) and a physical property measurement system (PPMS, available from Quantum Design). The appended FIG. 7 is a graph of magnetization value versus temperature (M-T) of a magnetic nanoparticle according to one embodiment as disclosed herein at 100 Oe. As illustrated in FIG. 7, the magnetic nanoparticle (La_{0.75}Sr_{0.25}(MnO₃)₁) as disclosed herein comprising a rare earth metal, a divalent metal, and a transition metal oxide had a magnetization value of 0 at temperatures equal to and above 310 K (37°C).

## Claims

1. A composition for target substance detection comprising:
a magnetic nanoparticle having a Curie temperature within the range of 0°C to 41°C, comprising a lanthanum metal, a strontium metal, and manganese oxide, and consisting of 0.5 to 1 molar fraction of the lanthanum metal and 0.01 to 0.5 molar fraction of the strontium metal relative to 1 molar fraction of the manganese oxide; and
a composite consisting of a magnet and an antibody;
wherein the magnet is a conductive material.

2. The composition according to claim 1, wherein a detection means is attached to a surface of the magnetic nanoparticle.

3. The composition according to claim 2, wherein the detection means is a fluorescent material or a quantum dot.

4. The composition according to claim 3, wherein the fluorescent material is at least one selected from the group consisting of rhodamine, fluorescein, coumarin, acridine, pyrene, erythrosine, eosin, and 4-acetamido-4'-isothiocyanatostilbene-2,2'-disulfonic acid.

5. The composition according to claim 1, wherein the target substance is at least one selected from the group consisting of a protein, a DNA, and a RNA.

6. The composition according to claim 5, wherein the protein is at least one selected from the group consisting of prostate specific antigen (PSA), carcinoembryonic antigen (CEA) MUC1, alpha fetoprotein (AFP), carbohydrate antigen 15-3 (CA 15-3), carbohydrate antigen 19-9 (CA 19-9), carbohydrate antigen 125 (CA 125), free prostate specific antigen (PSAF), prostate specific antigen-a 1-anticymotrypsin comple (PSAC), prostatic acid phosphatase (PAP), human thyroglobulin (hTG), human chorionic gonadotropin beta (HCGb), ferritin (Ferr), neuron specific enolase (NSE), interleukin 2 (IL-2), interleukin 6 (IL-6), beta 2 macroglobulin (B2M), and alpha 2 macroglobulin (A2M).

## Patentansprüche

1. Eine Zusammensetzung zum Zielsubstanznachweis, umfassend:
ein magnetisches Nanoteilchen mit einer Curie-Temperatur im Bereich von 0°C bis 41°C, umfassend ein Lanthanmetall, ein Strontiummetall und Manganoxid, und bestehend aus 0,5 bis 1 molarer Fraktion des Lanthanmetalls und 0,01 bis 0,5 molarer Fraktion des Strontiummetalls relativ zur 1-molaren Fraktion des Manganoxids;
und einen Verbund bestehend aus einem Magneten und einem Antikörper;
wobei der Magnet ein leitfähiges Material ist.

2. Die Zusammensetzung nach Anspruch 1. wobei ein Nachweismittel an einer Oberfläche des magnetischen Nanopartikels angeheftet ist.

3. Die Zusammensetzung nach Anspruch 2, wobei das Nachweismittel ein fluoreszierendes Material oder ein Quantenpunkt ist.

4. Die Zusammensetzung nach Anspruch 3, wobei das fluoreszierende Material mindestens eines ist, ausgewählt aus der Gruppe bestehend aus Rhodamin, Fluorescein, Cumarin, Acridin, Pyren, Erythrosin, Eosin und 4-Acetamido-4'-isothiocyanatostilben-2,2'-Disulfonsäure.

5. Die Zusammensetzung nach Anspruch 1, wobei die Zielsubstanz mindestens eine ist, ausgewählt aus der Gruppe bestehend aus einem Protein, einer DNA und einer RNA.

6. Die Zusammensetzung nach Anspruch 5, wobei das Protein mindestens eines ist, ausgewählt aus der Gruppe bestehend aus Prostata-spezifischem Antigen (PSA), karzinoembryonalem Antigen (CEA) MUC1, Alpha-Fetoprotein (AFP), Kohlenhydrat-Antigen 15-3 (CA 15-3), Kohlenhydrat-Antigen 19-9 (CA 19-9), Kohlenhydrat-Antigen 125 (CA 125), freiem Prostata-spezifischem Antigen (PSAF), Prostata-spezifischem Antigen-a 1-Antichymotrypsin-Komplex (PSAC), Prostata-spezifischer saurer Phosphatase (PAP), humanem Thyreoglobulin (hTG), humanem Choriongonadotropin beta (HCGb), Ferritin (Ferr), Neuronen-spezifischer Enolase (NSE), Interleukin 2 (IL-2), Interleukin 6 (IL-6), Beta-2-Makroglobulin (B2M) und Alpha-2-Makroglobulin (A2M).

## Revendications

1. Composition pour la détection d'une substance cible comprenant :
une nanoparticule magnétique ayant une température de Curie dans la plage de 0 °C à 41 °C, comprenant du métal lanthane, du métal strontium, et de l'oxyde de manganèse, et consistant en 0,5 à 1 fraction molaire du métal lanthane et 0,01 à 0,5 fraction molaire du métal strontium rapporté à 1 fraction molaire de l'oxyde de manganèse ; et
un composite consistant en un aimant et un anticorps ;
dans laquelle l'aimant est un matériau conducteur.

2. Composition selon la revendication 1, dans laquelle un moyen de détection est attaché à une surface de la nanoparticule magnétique.

3. Composition selon la revendication 2, dans laquelle le moyen de détection est un matériau fluorescent ou un point quantique.

4. Composition selon la revendication 3, dans laquelle le matériau fluorescent est au moins l'un choisi dans le groupe consistant en la rhodamine, la fluorescéine, la coumarine, l'acridine, le pyrène, l'érythrosine, l'éosine, et l'acide 4-acétamido-4'-isothiocyanatostilbène-2,2'-disulfonique.

5. Composition selon la revendication 1, dans laquelle la substance cible est au moins l'un choisi dans le groupe consistant en une protéine, un ADN et un ARN.

6. Composition selon la revendication 5, dans laquelle la protéine est au moins l'un choisi dans le groupe consistant en un antigène spécifique de la prostate (PSA), l'antigène carcino-embryonnaire (CEA) MUC1, l'alpha-foetoprotéine (AFP), l'antigène glucidique 15-3 (CA 15-3), l'antigène glucidique 19-9 (CA 19-9), l'antigène glucidique 125 (CA 125), l'antigène spécifique de la prostate libre (PSAF), le complexe antigène spécifique de prostate-α1-antichymotrypsine (PSAC), la phosphatase acide prostatique (PAP), la thyroglobuline humaine (hTG), la gonadotrophine chorionique bêta humaine (HCGb), la ferritine (Ferr), l'énolase spécifique des neurones (NSE), l'interleukine 2 (IL-2), l'interleukine 6 (IL-6), la macroglobuline bêta 2 (B2M), et la macroglobuline alpha 2 (A2M).
